# EUROPÄISCHE PATENTSCHRIFT

(11) **EP 1 359 415 B1**
(45) Veröffentlichungstag und Bekanntmachung des Hinweises auf die Patenterteilung: **17.12.2003**
(21) Anmeldenummer: 02009903.2
(22) Anmeldetag: 03.05.2002
(51) Int. Cl.: G01N 33/50, G01N 33/487, G01N 21/07

(54) **Vorrichtung und Verfahren zum Lysieren von Zellen**
Device and process to lyse cells
Dispostiv et procédé de la lyse des cellules

(43) Veröffentlichungstag der Anmeldung: 05.11.2003
(73) Patentinhaber: Trace Biotech AG, 38106 Braunschweig (DE)
(72) Erfinder: Wieland, Martin, Dr., 38723 Seesen (DE); Heinsch, Stefan, 38122 Braunschweig (DE)
(74) Vertreter: Eisenführ, Speiser & Partner

(56) Entgegenhaltungen:
- WO-A-97/21090
- WO-A-99/35499
- GB-A- 2 355 717
- US-A- 6 106 779

## Beschreibung

Die Erfindung betrifft eine Vorrichtung und ein Verfahren zum Lysieren von Zellen. Die Erfindung betrifft des weiteren Assay-Anordnungen und Verfahren zur Durchführung eines Assays (Tests), wobei jeweils eine erfindungsgemäße Lysier-Vorrichtung oder ein erfindungsgemäßes Lysier-Verfahren Einsatz finden.

Die sogenannte Point-of-Care-Untersuchung von biologischen Proben ist im Bereich der Medizin von zunehmender Bedeutung; sie soll z.B. niedergelassenen Ärzten und Ambulanzpersonal ohne Zwischenschaltung biologisch-technischer Laboratorien die Durchführung medizinischer Assays und eine kostengünstige Diagnose ermöglichen.

Die Tests sollen dabei einfach und bequem durchzuführen sein und schnell zu reproduzierbaren Ergebnissen führen. Insbesondere im Gebiet der Notfall-Medizin, aber auch z.B. im Rahmen der üblichen Praxis eines niedergelassenen Arztes werden die Testergebnisse häufig sofort benötigt und haben die behandelnden Ärzte oder Sanitäter nur sehr wenig Zeit, den Test durchzuführen. Im Zusammenhang mit einer Vielzahl diagnostisch wertvoller Assays ist eine Aufbereitung oder Vorbereitung der zu untersuchenden biologischen Probe erforderlich. In diesem Fällen ist es von enormer Wichtigkeit, dass der Schritt der Probenauf- bzw. -vorbereitung schnell, komfortabel und einfach durchführbar ist und zu reproduzierbaren Resultaten führt. Der Auf- bzw. Vorbereitungsschritt ist dabei aber nicht isoliert von den weiteren Verfahrensschritten eines Point-of-Care-Assays zu betrachten, sondern als ein wesentlicher Teil eines solchen Assays, der mit den vor- und nachgeschalteten Verfahrensschritten möglichst kompatibel und durch definierte zeitlich-räumliche Transferpunkte verknüpft sein sollte.

Für die Untersuchung von fluiden biologischen Proben, die zelluläres Material umfassen, ist es häufig notwendig, das Zellmaterial zu lysieren und erst im Anschluss an diesen Präparationsschritt (Probenvorbereitungsschritt) die resultierende Flüssigkeit zu untersuchen. Bei der Probe kann es sich beispielsweise um eine Blut- oder Urinprobe handeln, die z.B. auf Stoffwechselverbindungen oder wichtige physiologische Kontrollparameter wie z.B. Hämoglobin oder Hämoglobin A1c (HbA1c) untersucht werden sollen. Im Anschluss an den Lyse-Schritt wird es häufig auch erforderlich sein, die bei der Lyse entstehenden Zellmembranpartikel (Debris) von der Probenflüssigkeit abzutrennen, die nach der Lyse aus extrazellulärer und intrazellulärer Flüssigkeit (inklusive der jeweiligen gelösten Substanzen) besteht.

Befindet sich der Analyt eines durchzuführenden Assays innerhalb des zellulären Materials einer biologischen Probe, ist zur Vorbereitung des Assays regelmäßig das Lysieren des Zellmaterials erforderlich. Neben den bereits erwähnten Stoffwechselverbindungen und physiologischen Kontrollparametern kann es sich bei den jeweiligen Analyten auch um sonstige zelluläre Inhaltsstoffe handeln, wie z.B. Medikamente oder deren Abbauprodukte.

Es sind bereits Vorrichtungen und Verfahren zum Lysieren zellulären Materials in einer biologischen Probe und zum Abtrennen von festen Bestandteilen, die nach dem Lysieren vorliegen, bekannt.

So offenbart die US 6,106,779 (Büchler et al) Assay-Einrichtungen mit einer Lysierkammer, die lysierendes Material auf einer Oberfläche und ein Sieb (mesh) umfasst, auf dem lysierendes Material angeordnet sein kann. Die Lysierkammer steht in Fluidverbindung mit einem Probenaufgabebereich und einem Probenausgangsport (fluid egress port), an den sich ein Ausgangsbereich (exit region) anschließt. Der Ausgangsbereich kann eine oder mehr Komponenten eines Systems zur Durchführung eines Assays umfassen. Zum Erreichen eines Fluidtransports zwischen der Lysierkammer und dem Ausgangsbereich wird vorgeschlagen, dass der Ausgangsbereich eine gleiche oder höhere Kapillarität im Vergleich mit der Lysierkammer besitzt. Aufgrund dieser Kapillaritätsverhältnisse verlässt eine Probenflüssigkeit die Lysierkammer ohne einen äußeren Druck und füllt den Kapillarraum des Ausgangsbereiches gleichförmig aus. Es werden gemäß der US 6,106,779 auch vorteilhafterweise Kapillarkräfte eingesetzt, um eine Probenflüssigkeit in die Lysierkammer hinein zu transportieren.

Bei Verwendung der in der US 6,106,779 beschriebenen Verfahren erscheint es jedoch nachteilig, dass häufig relativ große Mengen an Probenflüssigkeit in der Lysierkammer und den Kanälen des Systems zurückbleiben und für den eigentlichen Test nicht zur Verfügung stehen; zudem ist die Reproduzierbarkeit des Produktes einer Probenvorbereitung unter Verwendung der in der US 6,106,779 beschriebenen Einrichtung häufig nicht ausreichend. In der US 6,106,779 selbst wird auf die vielfältigen Möglichkeiten der "performance tunability of a lysis chamber" hingewiesen (vergleiche Spalte 8, Zeile 65 bis Spalte 10, Zeile 58), und augenscheinlich reichen bereits kleinste (zufällige) Unterschiede in der Ausgestaltung der Lysierkammer oder der mit dieser in Fluidverbindung stehenden Systemkomponenten aus, um bei der Vorbereitung (Lyse; Abtrennen von Zellmembranpartikeln) identisch zusammengesetzter Blutproben unterschiedliche Ergebnisse zu erhalten. Die Unterschiede betreffen dabei insbesondere die Parameter (a) Menge aufbereiteter Probenflüssigkeit bezogen auf die eingesetzte Menge der Blutprobe und (b) Viskosität der Probenflüssigkeit nach Austritt aus der Lysierkammer.

Die US 5,242,842 beschreibt einen Assay für glykiertes Hämoglobin, zu dessen Vorbereitung eine Hämolyse der Erythrozyten in einer Blutprobe vorgenommen werden kann. Die US 5,242,842 betrifft jedoch keine Vorrichtung und kein Verfahren, das zum Point-of-Care-Einsatz geeignet wäre.

US 4,987,085 beschreibt Verfahren zum Abtrennen roter Blutzellen aus einer Blutprobe, wobei die Hämolyse dieser Zellen weitestgehend vermieden werden soll.

US 6,046,019 offenbart Verfahren zum Testen auf die Anwesenheit von Granulozyten in Blut, wobei rote Blutzellen lysiert und die Debris der lysierten roten Blutzellen von den (nicht lysierten) Granulozyten abgetrennt werden. Das offenbarte Verfahren erfordert einen relativ hohen manuellen Aufwand und ist deshalb für den Point-of-Care-Einsatz nur bedingt tauglich.

US 4,435,505 offenbart ein Verfahren, in dem in einer ersten Kammer einer für medizinische Point-of-Care Anwendungen zu großen Laborapparatur die Lyse einer Blutprobe durchgeführt wird und die lysierte Probenflüssigkeit dann vakuumunterstützt über ein Filter in eine darunter gelegene zweite Kammer transportiert wird, wobei der Filter feste Bestandteile der lysierten Blutprobe zurückhält, also insbesondere Membranpartikel roter und weißer Blutzellen.

US 6,251,660 B1 offenbart Verfahren zur Untersuchung von Körperflüssigkeiten wie Blut, Urin, Speichel oder Rückenmarksflüssigkeit, wobei die Lyse von Zellen zur Vorbereitung des eigentlichen Untersuchungsschrittes vorgesehen sein kann. Die beschriebene Untersuchungsanordnung umfasst eine zylindrische Röhre mit voneinander durch zerreißbare Membranen getrennten Kammern und erscheint für den Point-of-Care-Einsatz allenfalls bedingt tauglich.

Zudem ist aus der GB 2,355,717A ein Verfahren zur DNA-Isolierung sowie eine entsprechende Vorrichtung bekannt. Die Vorrichtung umfasst dabei eine rotierbare Plattform mit einer flachen Scheibe, die ein Zentrum besitzt, um weiches die Plattform rotierbar ist. Ein Lysat biologischer Zellen kann in eine Kammer eintreten, welche ein Filtermaterial besitzt, auf dem die im Lysat enthaltene DNA und/oder Zellkerne zurückgehalten werden. Andere Bestandteile des Lysats passieren das Filtermaterial jedoch weitgehend ungehindert. Verschiedene Waschschritte und ein weiterer Lyseschritt zur Freisetzung zusätzlicher DNA aus den gefangenen Zellkernen werden angegeben, um die DNA-Isolierung durchzuführen. Eine Abtrennung von Zellmembranpartikein von den sonstigen Inhaltsstoffen in der Zelle (mit Ausnahme der DNA und der Zellkerne) wird nicht beschrieben. Es war eine Aufgabe der vorliegenden Erfindung, eine Vorrichtung und ein Verfahren zum Lysieren von Zellen anzugeben, die insbesondere zur Verwendung im Point-of-Care-Bereich geeignet und als solche leicht handhabbar bzw. durchführbar sein sollten.

Vorrichtung bzw. Verfahren sollten vorzugsweise neben der Lyse auch die Trennung einzelner oder sämtlicher Inhaltsstoffe einer (lysierten) biologischen Zelle von Zellmembranpartikein ermöglichen, die beim Lysieren der Zelle entstehen.

Die Ergebnisse einer in der anzugebenden Vorrichtung bzw. mittels des anzugebenden Verfahrens durchgeführten Lyse (gegebenenfalls Abtrennung von Zellmembranpartikeln) sollten überdies gut reproduzierbar sein.

Es sollten zudem vorteilhafterweise klare zeitliche und räumliche Transferpunkte für den Übergang von (a) vorgelagerten Verfahrensschritten (bzw. den entsprechenden Orten innerhalb der Vorrichtung) zu (b) den Schritten der Lyse und gegebenenfalls Abtrennung sowie von (b) zu (c) den nachgelagerten Verfahrensschritten (bzw. den entsprechenden Orten in der Vorrichtung) vorhanden sein.

Mit Blick auf die anzugebende Vorrichtung werden die gestellten Aufgaben erfindungsgemäß gelöst durch eine Vorrichtung zum Lysieren von biologischen Zellen, umfassend:
- eine rotierbare Plattform, umfassend eine flache Scheibe (z.B. von kreisrunder, ovaler oder polygonaler Form), die ein Zentrum besitzt, um welches die Plattform rotierbar ist (und deren Schwerpunkt vorzugsweise im Zentrum lokalisiert ist, um eine Rotation auch bei hohen Rotationsgeschwindigkeiten sicher zu ermöglichen),
- eine erste Kammer, umfassend eine Vertiefung in der flachen Scheibe mit einem Volumen im Bereich zwischen 0,1 µl und 100 µl,
- eine zweite Kammer, umfassend eine Vertiefung in der flachen Scheibe mit einem Volumen im Bereich zwischen 0,1 µl und 100 µl, wobei die zweite Kammer in radialer Richtung weiter vom Zentrum entfernt positioniert ist als die erste Kammer,
- einen ersten Kanal in der flachen Scheibe, der die erste und die zweite Kammer verbindet und so eingerichtet und angeordnet ist, dass ein zentripetalkraftunterstützter Transport einer Probenflüssigkeit von der ersten zu der zweiten Kammer durch Rotation der Plattform um das Zentrum möglich ist,
wobei die erste Kammer umfasst:
- ein Filtermaterial, das in der ersten Kammer so angeordnet ist, dass in die erste Kammer eingebrachte Probenflüssigkeit bei Rotation der Plattform eine Kraft in Richtung auf das Filtermaterial und/oder aus dem Filtermaterial heraus in Richtung auf den ersten Kanal erfährt, und
- ein Reagenz zum Lysieren von biologischen Zellen, das auf dem Filtermaterial angeordnet ist.

Als flache Scheibe, welche ein Zentrum besitzt, um welches die Plattform rotierbar ist, können insbesondere kreisrunde Scheiben nach Art einer optischen Disk eingesetzt werden, wie sie als Compact Disc (CD; CD-ROM), Digital Versatile Disc (DVD) oder Mini Disc bekannt sind. Die weiteren strukturellen Merkmale der erfindungsgemäßen Vorrichtung (wie Probeneinlass, erste Kammer, zweite Kammer, erster Kanal, Filtermaterial usw.) lassen sich auf an sich bekannte Weise in die flache Scheibe integrieren.

Rotierbare Plattformen, die eine flache Scheibe umfassen, welche ein Zentrum besitzt, um das die Plattform rotierbar ist, wobei die flache Scheibe einen Probeneinlass, eine erste und eine zweite Kammer und einen oder mehrere Verbindungskanäle besitzt, sind aus dem Stand der Technik bereits seit längerem bekannt. Es wird auf folgende Schriften verwiesen, deren Offenbarungsgehalt im Wege der Verweisung Bestandteil dieser Anmeldung ist, soweit die Ausgestaltung einer rotierbaren Plattform inklusive der Gestaltung der darin enthaltenen (mikro-)fluidischen Strukturen betroffen ist:

US 4,279,862 (Bretaudiere et al.), die einen "centrifugal photometric analyzer" betrifft.

US 4,225,558 (Peterson et al.), betreffend "fluid sample test apparatus and fluid sample cell for use therein".

US 4,237,234 (Meunier), betreffend ein "device for use in the study of biochemical or enzymatic reactions produced by living organisms".

US 3,795,451 (Mailen), betreffend einen "rotor for fast analyser of rotary cuvette type".

US 3,829,223 (Hamel), betreffend einen "mixing rotor for fast analyser of rotary cuvette type with means for enhancing the mixing of sample and reagent liquids".

US 4,123,173 (Bullock et al.), betreffend "rotatable flexible cuvette arrays".

US 4,154,793 (Guigan), betreffend ein "device for conditioning a sample of liquid for analysing".

US 3,555,284, betreffend ein "multistation, single channel analytical photometer and method of use".

US 6,063,589 (Kellogg et al.), betreffend "devices and methods for using centripetal acceleration to drive fluid movement on a microfluidics system". Dieses Dokument beschreibt neben der bauartlichen Ausgestaltung einer rotierbaren Plattform für die Probenpräparation auch eine Blut-Auftrennungsanordnung. Eine Lyse von Blutzellen wird jedoch vermieden.

WO 97/21090 (Gamera Bioscience), betreffend "devices and methods for using centripetal acceleration to drive fluid movement in a microfluidics system with on-board informatics".

US 6,030,581 (Virtanen), betreffend ein "laboratory in a disk". Diese Schrift offenbart Verfahren zur Aufreinigung von Flüssigkeiten auf einer optischen Disk; die Lyse von Zellen im Rahmen eines Bromverbrennungsverfahrens wird jedoch nicht beschrieben.

WO 99/35499 (Remacle, José), betreffend eine "method comprising capture molecule fixed on disc surface".

WO 97/08556 (First Medical, Inc.), betreffend einen "analytical rotor and method for detecting analytes in liquid samples". Diese Schrift offenbart unter anderem ein Verfahren zur Bestimmung des Hämatokrit-Wertes im Blut; ein Hinweis auf eine Probenvorbereitung, bei der Blutzellen lysiert werden, ist der Schrift jedoch nicht zu entnehmen.

US 5,061,381 (Burd), betreffend einen "apparatus and method for separating cells from biological fluids".

US 5,693,233 (Schembri), betreffend "methods of transporting fluids within an analytical rotor". In dieser Schrift wird unter anderem die Auftrennung von Blut in Plasma und zelluläres Material beschrieben. Ein Probenvorbereitungsverfahren, welches die Lyse von Zellen innerhalb einer biologischen Flüssigkeit umfasst, wird jedoch nicht beschrieben.

Zum technologischen Hintergrund der vorliegenden Erfindung zählen auch die folgenden Schriften:
US 5,122,284, US 5,186,844, US 5,173,193, US 5,412,732, WO 98/13684.

Das in der ersten Kammer einer erfindungsgemäßen Vorrichtung angeordnete Filtermaterial ist vorteilhafter Weise dazu eingerichtet, Zellmembranpartikel, die beim Lysieren einer biologischen Zelle entstehen, bei Rotation der Plattform in der ersten Kammer zurückzuhalten. Vorteilhafterweise ermöglicht es gleichzeitig den Rückhalt von gegebenenfalls nicht lysierten Zellen, so dass bei Einsatz der erfindungsgemäßen Vorrichtung das anfallende Filtrat (die zur Untersuchung vorbereitete Probe) zumindest weitestgehend von Feststoffen befreit ist. Als Filtermaterialien sind generell alle üblichen Filtermaterialien geeignet, die in der Lage sind, die anfallenden Zellbestandteile bzw. nicht-lysierten Zellen festzuhalten. Besonders gute Separationsergebnisse lassen sich mit Glasfiltermaterialien erzielen, die gesintert sind oder durch geeignete Bindemittel untereinander vernetzt sind. Als poröse Filtermaterialien können aber ebenfalls Materialien aus diversen Kunststoffen eingesetzt werden, wie z.B. Polysulfonfilter, Nylon-Filter, Filter aus fluor-organischen Materialien wie Teflon, Filter aus Polyethylen, Polyamid oder Polypropylen. Auch Filtermaterialien, die aus Naturstoffen gewonnen werden, können eingesetzt werden, beispielsweise Papierfilter, Filter aus Celluloseacetat oder -butyrat sowie Filter aus Nitrocellulose. Auch Filter aus Metall oder Keramik können vorteilhaft eingesetzt werden. Die Verwendung von Kombinationen aus verschiedenen Filtermaterialien kann im Einzelfall vorteilhaft sein. Das Filtermaterial kann als lateraler oder transversaler Filter eingesetzt werden, wobei die Ausgestaltung als lateraler Filter bevorzugt ist. Kombinationen aus lateralen und transversalen Filtern sind in Einzelfällen vorteilhaft.

Vorteilhafterweise ist das Filtermaterial so ausgestaltet, dass Partikel eines Durchmessers von 0,2 oder mehr µm zurückgehalten werden (Ausschlussgröße). Vorzugsweise liegt die Ausschlussgröße im Bereich eines Partikeldurchmessers von 0,3 bis 5 µm und weiter vorzugsweise im Bereich zwischen 0,5 und 4,5 µm. Am bevorzugtesten ist eine Ausschlussgröße im Bereich zwischen 1 und 3 µm Partikeldurchmesser. Selbstverständlich können Filtermaterialien mit unterschiedlichen Porengrößen (Ausschlussgrößen) kombiniert werden, um das Trennergebnis bei der durch Rotation der Plattform verursachten Filterzentrifugation (zentripetalkraft-unterstützten Filtration) zu optimieren.

Hinsichtlich der Auswahl des Filtermaterials sei an dieser Stelle erneut auf die US 6,106,779 (Büchler et al) verwiesen, die sich intensiv mit den Aspekten "Lysis Mesh/Matrix", "Mesh Material", "Mesh Filament and Mesh Weave", "Mesh Pore Size", "Mesh Cutting", "Mesh Treatment" and "Mesh Attachment" (Spalten 5 und 6) befasst. Die hinsichtlich der besagten Aspekte in der US 6,106,779 angegebenen bevorzugten Ausgestaltungen gelten im Rahmen der vorliegenden Erfindung entsprechend und sind im Wege der Verweisung Bestandteil dieser Anmeldung.

Das Filtermaterial ist vorzugsweise dazu eingerichtet, sämtliche Zellmembranpartikel, die beim Lysieren einer biologischen Zelle gebildet werden, bei Rotation der Plattform in der ersten Kammer zurückzuhalten; es ist deshalb eine quantitative Trennung von Zellmembranpartikeln und Probenflüssigkeit (als Filtrat der bei Rotation der Plattform stattfinden Filterzentrifugation) möglich.

Als Reagenz zum Lysieren von biologischen Zellen, das in einer erfindungsgemäßen Vorrichtung auf dem Filtermaterial angeordnet ist, können die üblichen Reagenzien eingesetzt werden. Geeignet sind insbesondere alkalische Substanzen (Basen), Detergenzien (oberflächenaktive Chemikalien) wie z.B. Octanoyl-N-methylglucamin (Mega 8), t-Octylphenoxypolyethoxyethanol (Triton-x-100), Laurylsulfat-Salze (insbesondere Natrium-, Ammonium- und Triethanolamin-Salze), alpha-olefinische Sulfonate, Laurylamidopropyldimethylaminoxid, Lauryldiethanolamid, Lauryldimethylaminoxid, Betain, Amidobetain Glycinat, Sulfobetain, Stearyldimethylbenzylammoniumchlorid und weitere Derivate von Ammoniumsalzen, insbesondere quartären Ammoniumsalzen. Es können auch ionische (insbesondere anionische) und nichtionische Tenside eingesetzt werden, insbesondere Derivate von Polyoxyethylen. Auch Säuren wie Ameisensäure und Essigsäure können als Lysier-Reagenz verwendet werden. Ionische Substanzen wie Cyanide und Lithiumsalze (z.B. Lithiumthiocyanat) sind ebenfalls als Lysier-Reagenz einsetzbar. Auch Saponin und andere natürlich vorkommende Stoffe mit der Befähigung zur Lyse von Zellen können in der erfindungsgemäßen Vorrichtung eingesetzt sein. Das Lysierreagenz kann insbesondere als Feststoff oder Gel vorliegen.

Unter den vorgenannten und weiteren Reagenzien zum Lysieren von biologischen Zellen wird der Fachmann dasjenige Reagenz auswählen, welches im jeweiligen Einzelfall (also abhängig von den zu lysierenden Zellen und der zu präparierenden Probe) am geeignetsten erscheint. Auch Mischungen der vorgenannten Reagenzien untereinander oder mit sonstigen Reagenzien können insoweit eingesetzt werden und werden dann als "Reagenz zum Lysieren von biologischen Zellen" bezeichnet.

Gemäß einer besonders bevorzugten Ausgestaltung der erfindungsgemäßen Vorrichtung sind der ersten Kammer und/oder dem ersten Kanal Rückhaltemittel zugeordnet, die den Transport einer (gegebenenfalls aufbereiteten) Flüssigkeitsprobe von der ersten Kammer in die zweite Kammer verhindern oder behindern, wenn die Plattform (a) nicht rotiert oder (b) mit einer Geschwindigkeit rotiert, die unterhalb eines vorbestimmten Schwellenwertes liegt. Dieses Rückhaltemittel kann beispielsweise eine Kapillarsperre sein, wie sie (in anderem technischen Zusammenhang) z.B. aus der US 6,063,589 (Kellogg et al) bekannt ist. Eine Kapillarsperre (capillary barrier) verhindert den Transport (Fluss) einer Flüssigkeitsprobe bei einer ersten Rotationsgeschwindigkeit F₁, gestattet jedoch den Transport (Fluss) der Flüssigkeitsprobe bei einer zweiten Rotationsgeschwindigkeit F₂, wenn gilt: F₂ > F₁. Rotiert also die Plattform mit einer Geschwindigkeit F₂, so wird eine Flüssigkeitsprobe von der ersten Kammer durch den ersten Kanal in die zweite Kammer transportiert, rotiert die Plattform jedoch mit einer geringeren Geschwindigkeit F₁, so verbleibt die Flüssigkeitsprobe (zumindest im Wesentlichen, vorzugsweise jedoch vollständig) in der ersten Kammer.

Anstelle einer Kapillarsperre können drehzahlgesteuerte Mikroventile oder dergleichen als Rückhaltemittel vorgesehen werden.

Vorteilhaft ist es auch, wenn eine erfindungsgemäße Vorrichtung eine Schalteinrichtung umfasst, um eine Rotation der Plattform mit einer Geschwindigkeit auszulösen, die oberhalb eines vorbestimmten Schwellenwertes liegt, sobald eine Flüssigkeitsprobe für einen vorbestimmten Zeitraum in der ersten Kammer verweilt hat.

Wenn Rückhaltemittel der beschriebenen Art in der erfindungsgemäßen Vorrichtung vorgesehen sind, lässt sich besonders mühelos eine Probenflüssigkeit für einen definierten Zeitraum in der ersten Kammer halten, z.B. solange (a) bis zumindest 50 % der Zellen eines ausgewählten Zelltyps (der in der Flüssigkeit enthalten ist) lysiert sind, oder bis (b) die Lyse dieses ausgewählten Zelltyps oder (c) sämtlicher in der Flüssigkeitsprobe vorhandener Zellen komplettiert ist. Das Trennen der Probenflüssigkeit nach dem Lyseschritt von den dann in der Probenflüssigkeit enthaltenen Feststoffen (insbesondere Zellmembranpartikeln, gegebenenfalls jedoch auch nicht-lysierten Zellen sowie zufälligen Verunreinigungen) lässt sich bei einer derartigen Ausgestaltung in einem separaten Verfahrensschritt durchführen. Die Verfahrensschritte (a) Lyse und (b) Abtrennen der Probenflüssigkeit von festen Inhaltsstoffen können somit nacheinander erfolgen, was zu gut reproduzierbaren Ergebnissen der Probenvorbereitung (und gegebenenfalls einer nachfolgenden Analyse) führt.

Alternativ können die Schritte (a) Lyse und (b) Abtrennen (= Filtration) auch simultan durchgeführt werden.

Zur Herstellung der erfindungsgemäßen Vorrichtungen können die erforderlichen Fluidikstrukturen (Kammern, Kanäle, etc.) mittels üblicher mikrotechnischer Verfahren z.B. durch photolithographische Prozesse, Fräsen, Verwendung von Lasern, Heißprägen, Spritzgießen z.B. auf eine Polycarbonat Compact Disc oder einen anderen Kunststoffträger aufgebracht werden. Gegenüber der Compact Disc-Oberfläche besitzen übliche Kanäle und Kammern eine Tiefe im Bereich von 100 bis 800 µm, vorzugsweise im Bereich zwischen 300 und 500 µm, typischerweise etwa 400 µm. (Verbindungs-)Kanäle zwischen Kammern besitzen regelmäßig eine Breite im Bereich zwischen 0,2 und 0,8 mm, typischerweise eine Breite von etwa 0,5 mm. Die Kammern besitzen eine maximale Breite im Bereich zwischen 2 und 10 mm, vorzugsweise zwischen 3 und 7 mm; üblicherweise werden Kammern mit einer kreisförmigen Grundform vorgesehen, wobei der Kammerdurchmesser im Bereich zwischen 2 und 5 mm liegt. Andere Geometrien sind auch möglich. Wie weiter unten noch näher ausgeführt, sind regelmäßig Entlüftungskanäle vorgesehen, und diese besitzen eine Kanalbreite im Bereich zwischen 50 und 500 µm, vorzugsweise im Bereich zwischen 150 und 250 µm, typischerweise etwa 200 µm. Das Fluidiksystem (Kanäle, Kammern etc.) ist üblicherweise durch eine flache Folie flüssigkeitsdicht verschlossen, wobei die Folie vorzugsweise eine transparente Klebefolie ist, welche auf die Oberfläche der Scheibe (z.B. Compact Disc) aufgeklebt ist. In der Folie sind Öffnungen für die Probenaufgabe und die Entlüftungskanäle (soweit vorhanden) vorgesehen.

Alternativ zu einem Verschluss mittels Folie ist auch eine Abdeckung mittels einer zweiten Scheibe der gleichen oder einer ähnlichen Geometrie möglich.

Für das Verschließen des Flüssigkeitssystems können alternativ zum Aufkleben auch andere geeignete Verfahren eingesetzt werden, z.B. Lösungsmittelbonden, Ultraschallverschweißen, thermisches Verschweißen, Laserschweißen, Klebeverfahren.

Die Erfindung betrifft auch ein Verfahren zum Lysieren von biologischen Zellen (und zum Trennen fester von flüssigen Produkten der Lyse), mit folgenden Schritten:
- Bereitstellen einer biologische Zellen eines einzelnen Zelltyps oder verschiedener Zelltypen enthaltenden Flüssigkeit, z.B. einer biologischen Flüssigkeit oder einer Körperflüssigkeit wie Blut, Urin, Auswurf (Sputum), Samenflüssigkeit, Speichel, Tränenflüssigkeit, Gehirnflüssigkeit, Magenflüssigkeit, Fruchtwasser, Rückenmarksflüssigkeit, eine Zellkulturflüssigkeit oder eine zellhaltige Nahrungsmittel- oder Industriechemikalie,
- Bereitstellen einer erfindungsgemäßen Vorrichtung,
- Einbringen eines Volumens der Flüssigkeit in die erste Kammer der Vorrichtung,
- Kontaktieren der Flüssigkeit in der ersten Kammer mit dem Reagenz zum Lysieren, bis ein vorbestimmter Anteil der Zellen eines ausgewählten Zelltyps lysiert ist, beispielsweise zumindest 50 % der Zellen des ausgewählten Zelltyps (Verfahrensschritt "fraktionierte Lyse"),
- Rotieren der Plattform um das Zentrum der Vorrichtung, so dass die Flüssigkeit aus der ersten Kammer (gegebenenfalls unter Passieren einer Kapillarsperre oder eines sonstigen Rückhaltemittels) durch den ersten Kanal in die zweite Kammer transportiert wird und zumindest ein Teil der Membranpartikel lysierter Zellen und/oder sonstige Feststoffe vom Filtermaterial in der ersten Kammer zurückgehalten werden (Verfahrensschritt: "Filterzentrifugation").

Die Schritte "fraktionierte Lyse" oder "Filterzentrifugation" können sowohl nacheinander (sequenziell) als auch zeitgleich (simultan) durchgeführt werden. Voraussetzung für eine simultane Durchführung ist lediglich, dass der Zeitraum des Kontaktes zwischen Flüssigkeit und Lysier-Reagenz ausreicht, um einen ausreichenden (vorbestimmten) Anteil der Zellen des ausgewählten Zelltyps zu lysieren. Schließt sich beispielsweise an die Schritte "fraktionierte Lyse" und "Filterzentrifugation" ein Analysenschritt beispielsweise in der zweiten Kammer an, für dessen erfolgreiche Durchführung lediglich ein äußerst geringer Anteil von Zellen lysiert werden muss, so steht einer simultanen Durchführung der beiden Verfahrensschritte "fraktionierte Lyse" und "Filterzentrifugation" nichts im Wege. In der Regel wird es jedoch vorteilhaft sein, die beiden Verfahrensschritte nacheinander (sequenziell) durchzuführen. In beiden Fällen besteht vorteilhafterweise die Möglichkeit, das Lysat/Filtrat in der zweiten Kammer unmittelbar im Anschluss z.B. photometrisch zu analysieren.

Weitere bevorzugte Ausgestaltungen des erfindungsgemäßen Verfahrens ergeben sich aus den vorteilhaften Ausgestaltungen der erfindungsgemäßen Vorrichtung, den Ansprüchen und der Beschreibung eines bevorzugten Ausführungsbeispiels weiter unten. Insbesondere kann es vorteilhaft sein, zur Überwindung einer gegebenenfalls vorgesehenen Kapillarsperre eine Rotationsgeschwindigkeit F₂ einzustellen, die größer ist als eine Rotationsgeschwindigkeit F₁, bei der der Transport einer Flüssigkeitsprobe von der ersten Kammer in die zweite Kammer (noch) verhindert oder behindert wird. Des weiteren ist es vorteilhaft, das erfindungsgemäße Verfahren so auszugestalten, dass die nach der Lyse vorliegenden flüssigen Bestandteile der eingesetzten Probe (inklusive flüssiger oder gelöster Bestandteile lysierter Zellen) von den Zellmembranpartikeln lysierter Zellen zumindest weitgehend abgetrennt werden; in der ersten Kammer verbleiben vorzugsweise nach dem Verfahrensschritt "Filterzentrifugation" maximal 20 Gew.-% (vorzugsweise zumindest 10 Gew.-%) der (lysierten) Probenflüssigkeit, d.h. werden insbesondere vom Filtermaterial und/oder gegebenenfalls vorhandenen Rückhaltemitteln zurückgehalten. Die Ausbeute (= Verhältnis Filtratmenge zur Menge eingesetzter Probenflüssigkeit) an Lysat/Filtrat wird somit insbesondere im Vergleich mit der Vorgehensweise gemäß der US 6,106,779 optimiert. Die Lyse-Anordnung ermöglicht wegen der hohen erreichbaren Ausbeuten zudem den Einsatz geringer Probenmengen.

Da die treibende Kraft für den Transport aus der ersten Kammer hinaus durch den ersten Kanal in die zweite Kammer drehzahlabhängig nahezu beliebig gesteuert werden kann, ermöglicht das erfindungsgemäße Verfahren auch den Transport höherviskoser Flüssigkeiten. Der Einsatz von Viskositätserniedrigern, wie er beispielsweise gemäß der US 6,106,779 (Büchler et al) praktiziert wird, ist daher im Rahmen der vorliegenden Erfindung zwar möglich, jedoch nur selten erforderlich. Bevorzugte erfindungsgemäße Verfahren sind die, in denen der zu untersuchenden Flüssigkeit keine viskositätsmodifizierenden (viskositätserniedrigenden) Stoffe zugesetzt werden, die somit auch in einer sich anschließenden Untersuchung nicht stören können.

Wie eingangs erwähnt, betrifft die vorliegende Erfindung auch eine Assay-Anordnung und ein Verfahren zur Durchführung eines Assays. Die erfindungsgemäße Assay-Anordnung umfasst dabei (a) eine erfindungsgemäße Vorrichtung sowie (b) Mittel zum Untersuchen einer Probenflüssigkeit in oder nach Entnahme aus der zweiten Kammer. Das erfindungsgemäße Verfahren zur Durchführung eines Assays umfasst sämtliche Schritte des erfindungsgemäßen Verfahrens zum Lysieren von Zellen sowie einen weiteren Schritt, in dem die lysierte und filterzentrifugierte Probenflüssigkeit untersucht wird. Es können dabei die üblichen Untersuchungs- und Analysemethoden eingesetzt werden.

Nachfolgend wird ein bevorzugtes Ausführungsbeispiel der vorliegenden Erfindung anhand der beigefügten Zeichnungen näher erläutert. Es stellt dar:

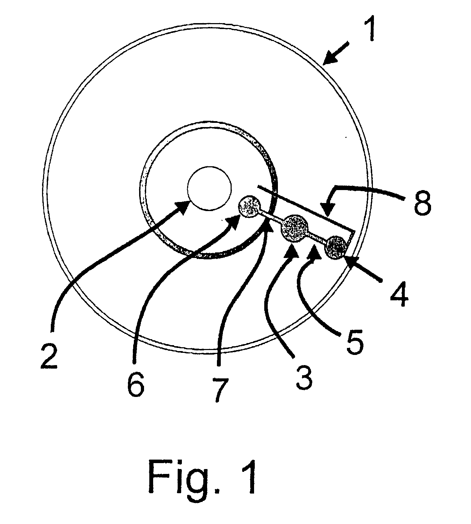

- Fig. 1: eine schematische Darstellung einer erfindungsgemäßen Vorrichtung zum Lysieren von Zellen.

Fig. 1 zeigt eine Vorrichtung zum Lysieren von biologischen Zellen mit einer rotierbaren Plattform, die eine flache Scheibe 1 umfasst. Diese flache Scheibe 1 besitzt CD- oder DVD-Format und umfasst ein Zentrum 2, um welches die Plattform (die flache Scheibe) in üblicher Weise rotierbar ist; das Zentrum ist dabei eine kreisrunde Ausnehmung in der Scheibe 1. Die Scheibe 1 besteht im Wesentlichen aus Polycarbonat, wie es zur Herstellung handelsüblicher CDs und DVDs eingesetzt wird.

Die Scheibe 1 umfasst eine erste Kammer 3 (Lysekammer), welche ein Volumen von ca. 2,5 µl besitzt und mittels eines photolithographischen Verfahrens in die Scheibe 1 eingebracht ist. Die Kammer 3 ist von im Wesentlichen kreisrunder Gestalt und besitzt einen Durchmesser von 4 mm.

In einem größeren radialen Abstand vom Zentrum 2 als die Kammer 3 liegt eine zweite Kammer 4 (Auffangkammer; Analysenkammer), die ebenso wie die Lysekammer 3 von im Wesentlichen kreisförmiger Gestalt ist und dieser auch hinsichtlich des Volumens und des Durchmessers entspricht. Ein erster Kanal 5 verbindet die Lysierkammer 3 mit der Auffangkammer 4. Kanal 5 besitzt eine Breite von 0,5 mm und eine Tiefe von 400 µm. Er gestattet den zentripetalkraft-unterstützten Transport einer wässrigen Probenflüssigkeit von der ersten Kammer 3 zu der zweiten Kammer 5 durch Rotation der Plattform (mit Scheibe 1) um das Zentrum 2 herum. Die Wandungen des ersten Kanals 5 sind hydrophob ausgerüstet, so dass ein Durchtritt einer wässrigen Probenflüssigkeit von der Lysierkammer 3 zur Auffangkammer zumindest erschwert, wenn nicht gar nicht ausgeschlossen ist, wenn die Scheibe 1 nicht um das Zentrum 2 herum mit einer ausreichenden Geschwindigkeit rotiert. Die hydrophobe Ausrüstung des Kanals 5 fungiert somit als Rückhaltemittel für eine in der Lysierkammer 3 vorliegende wässrige Flüssigkeit.

In die Lysierkammer 3 eingebettet befindet sich ein Filtermaterial (nicht näher dargestellt), welches die erste Kammer vollständig ausfüllt. Das Filtermaterial wurde vor dem Einlegen/Einbetten in die Lysierkammer 3 mit 2 µl eines Lysereagenzes (Saponin) in einer Konzentration von 5 mg/ml oder Mega 8 in einer Konzentration von 30 mg/ml getränkt und das in diesem Lysereagenz enthaltene Lösungsmittel (Wasser) vollständig abgedampft, so dass das Lysereagenz schließlich in rücklösbarer Form auf dem Filtermaterial angeordnet (fixiert) war.

Das Filtermaterial bestand aus handelsüblichem Material aus einem Verbundmaterial aus Naturfasern und Synthesefasern (Cytosep 6161, Pall Life Sciences).

In radialer Richtung näher am Zentrum positioniert als die Lysierkammer 3 befindet sich eine Probenaufgabekammer 6, welche über einen zweiten Kanal 7 mit der Lysierkammer 3 in Fluidverbindung steht. Kanal 7 ist dazu eingerichtet, eine Probenflüssigkeit allein unter der Wirkung von Kapillarkräften, d.h. auch in Abwesenheit unterstützender Zentripetalkräfte, aus der Probenaufgabekammer 6 in die Lysierkammer 3 zu transportieren. Die Probenaufgabekammer 6 entspricht hinsichtlich ihrer Dimension in etwa der Lysierkammer 3 und der Auffangkammer 4; der zweite Kanal 7 besitzt eine Breite von 500 µm und eine Tiefe von 400 µm.

In Verbindung mit der Auffangkammer 4 steht schließlich noch ein Entlüftungskanal 8, welcher ausgehend von der Auffangkammer 4 zunächst im rechten Winkel zum Kanal 5 verläuft, dann aber in Richtung auf das Zentrum 2 abknickt. Der Entlüftungskanal 8 besitzt einen Durchmesser von ca. 200 µm.

Mit Ausnahme der Probenaufgabekammer 6 und des freien Endes des Entlüftungskanals 8 ist das gesamte Fluidiksystem (Kanal 7, Lysierkammer 3, Kanal 5, Auffangkammer 4, Entlüftungskanal 8) gegenüber der Umgebung mit einer transparenten Klebefolie flüssigkeitsdicht verschlossen.

Zur Präparation einer Vollblut-Probe werden z.B. zwei Mikroliter des Vollblutes in die Probenaufgabekammer 6 hinein pipettiert. Die Blutprobe kann dabei unbehandelt oder heparinisiert eingesetzt werden. Aus der Kammer 6 zieht sich die Probe aufgrund der Kapillarwirkung des Kanales 7 automatisch in die Lysierkammer 3. Dieser Vorgang dauert mehrere Minuten.

In der Lysierkammer 3 steht die Blutprobe dann in Kontakt mit dem Filtermaterial und dem darauf angeordneten Lysierreagenz. Nach einer Einwirkzeit von etwa zwei bis drei Minuten sind die roten Blutzellen im Wesentlichen vollständig lysiert.

Die Scheibe 1 wird dann z.B. in einem üblichen CD-Laufwerk für 45 Sekunden oder kürzer rotiert, wobei das Lysat/Filtrat nach vollständigem Durchtritt durch das Filtermaterial durch den Kanal 5 hindurch (unter Überwindung der Sperrwirkung des Kanals) in die Auffangkammer 4 transportiert wird. Membranpartikel lysierter Zellen werden vollständig vom Filtermaterial zurückgehalten. Luft, die sich zunächst noch in Kanal 5 und Auffangkammer 4 befand, kann durch den Entlüftungskanal 8 entweichen. Das Lysat/Filtrat verbleibt schließlich in der Auffangkammer 4 und wird dort mittels einer nicht dargestellten Analyseneinrichtung untersucht.

Es sei angemerkt, dass die beschriebene Vorrichtung zum Lysieren von Zellen (bzw. die entsprechende Assay-Anordnung, die zusätzlich noch die Analysier-Einrichtung umfasst) in einer Vielzahl von Fällen bevorzugt ist, jedoch einzelne Vorrichtungsmerkmale abgewandelt werden oder entfallen können. Analoges gilt für die beschriebenen Verfahren.

## Patentansprüche

1. Vorrichtung zum Lysieren von biologischen Zellen, umfassend:
- eine rotierbare Plattform, umfassend eine flache Scheibe (1), die ein Zentrum (2) besitzt, um welches die Plattform rotierbar ist,
- eine erste Kammer (3), umfassend eine Vertiefung in der flachen Scheibe (1) mit einem Volumen im Bereich zwischen 0,1 µl und 100 µl,
- eine zweite Kammer (4), umfassend eine Vertiefung in der flachen Scheibe (1) mit einem Volumen im Bereich zwischen 0,1 µl und 100 µl, wobei die zweite Kammer (4) in radialer Richtung weiter vom Zentrum (2) entfernt positioniert ist als die erste Kammer (3),
- einen ersten Kanal (5) in der flachen Scheibe (1), der die erste und die zweite Kammer (3, 4) verbindet und so eingerichtet und angeordnet ist, dass ein zentripetalkraftunterstützter Transport einer Probenflüssigkeit von der ersten zu der zweiten Kammer (3, 4) durch Rotation der Plattform um das Zentrum (2) möglich ist,
wobei die erste Kammer (3) umfasst:
- ein Filtermaterial, das in der ersten Kammer (3) so angeordnet ist, dass in die erste Kammer (3) eingebrachte Probenflüssigkeit bei Rotation der Plattform eine Kraft in Richtung auf das Filtermaterial und/oder aus dem Filtermaterial heraus in Richtung auf den ersten Kanal (5) erfährt, und
- ein Reagenz zum Lysieren von biologischen Zellen, das auf dem Filtermaterial in rücklösbarer Form fixiert ist:

2. Vorrichtung nach Anspruch 1,
**dadurch gekennzeichnet, dass** das Filtermaterial dazu eingerichtet ist, Zellmembranpartikel, die beim Lysieren einer biologischen Zelle entstehen, bei Rotation der Plattform in der ersten Kammer (3) zurückzuhalten.

3. Vorrichtung nach einem der Ansprüche 1 oder 2,
**dadurch gekennzeichnet, dass** der ersten Kammer (3) und/oder dem ersten Kanal (5) ein Rückhaltemittel zugeordnet ist, das den Transport einer Flüssigkeitsprobe von der ersten Kammer (3) in die zweite Kammer (4) verhindert oder behindert, wenn die Plattform nicht rotiert oder mit einer Geschwindigkeit rotiert, die unterhalb eines vorbestimmten Schwellenwertes liegt
und/oder
eine Schalteinrichtung vorgesehen ist, um eine Rotation der Plattform mit einer Geschwindigkeit auszulösen, die oberhalb eines vorbestimmten Schwellenwertes liegt, sobald eine Flüssigkeitsprobe für einen vorbestimmten Zeitraum in der ersten Kammer (3) verweilt hat.

4. Verfahren zum Lysieren von biologischen Zellen, mit folgenden Schritten:
- Bereitstellen einer biologische Zellen eines einzelnen Zelltyps oder verschiedener Zelltypen enthaltenden Flüssigkeit,
- Bereitstellen einer Vorrichtung nach einem der Ansprüche 1 bis 3,
- Einbringen eines Volumens der Flüssigkeit in die erste Kammer (3) der Vorrichtung,
- Kontaktieren der Flüssigkeit in der ersten Kammer (3) mit dem Reagenz zum Lysieren, bis ein vorbestimmter Anteil der Zellen eines ausgewählten Zelltyps lysiert ist,
- Rotieren der Plattform um das Zentrum (2) der Vorrichtung, so dass die Flüssigkeit aus der ersten Kammer (3) durch den ersten Kanal (5) in die zweite Kammer (4) transportiert wird und zumindest ein Teil der Membranpartikel lysierter Zellen und/oder sonstige Feststoffe vom Filtermaterial in der ersten Kammer (3) zurückgehalten werden.

5. Verfahren nach Anspruch 4,
**dadurch gekennzeichnet, dass** die Plattform während des Kontaktierens der Flüssigkeit in der ersten Kammer (3) mit dem Reagenz zum Lysieren zumindest zeitweise nicht rotiert oder nur mit einer Geschwindigkeit rotiert wird, die keinen Transport von Flüssigkeit aus der ersten Kammer (3) durch den ersten Kanal (5) in die zweite Kammer (4) ermöglicht.

6. Verfahren nach Anspruch 4 oder 5,
**dadurch gekennzeichnet, dass** die Flüssigkeit für einen vorbestimmten Zeitraum in der ersten Kammer (3) mit dem Reagenz zum Lysieren kontaktiert wird.

7. Verfahren nach einem der Ansprüche 4 bis 6,
**dadurch gekennzeichnet, dass** die Plattform solange und mit einer solchen Geschwindigkeit um das Zentrum (2) der Vorrichtung rotiert wird, dass maximal 20 Gew.-% der Probenflüssigkeit nach dem Rotieren in der ersten Kammer (3) verbleiben.

8. Assay-Anordnung, umfassend eine Vorrichtung nach einem der Ansprüche 1 bis 3 sowie
- Mittel zum Untersuchen einer Probenflüssigkeit in oder nach Entnahme aus der zweiteri Kammer (4).

9. Verfahren zur Durchführung eines Assays, umfassend
- ein Verfahren zum Lysieren von biologischen Zellen nach einem der Ansprüche 4 bis 7 sowie
- Untersuchen der lysierten Probenflüssigkeit in oder nach Entnahme aus der zweiten Kammer (4).

10. Verwendung einer Vorrichtung nach einem der Ansprüche 1 bis 3 zum Lysieren von biologischen Zellen.

## Claims

1. Device for the lysis of biological cells, comprising:
- a rotatable platform, comprising a flat disc (1) which has a centre (2) about which the platform is rotatable,
- a first chamber (3), comprising a depression in the flat disc (1) having a volume in the range between 0.1 µl and 100 µl,
- a second chamber (4), comprising a depression in the flat disc (1) having a volume in the range between 0.1 µl and 100 µl,
the second chamber (4) being positioned farther from the centre (2) than the first chamber (3) in the radial direction,
- a first channel (5) in the flat disc (1) which connects the first and the second chambers (3, 4) and is so set up and arranged that transport of a sample liquid with the assistance of centripetal force is possible from the first to the second chamber (3, 4) as a result of the platform being rotated about the centre (2),
wherein the first chamber (3) comprises:
- a filter material which is so arranged in the first chamber (3) that, when the platform is rotated, sample liquid which is introduced into the first chamber (3) is subject to a force directed towards the filter material and/or out from the filter material towards the first channel (5) and,
- a reagent for the lysis of biological cells which is fixed to the filter material in re-releasable form.

2. Device according to claim 1, **characterised in that** the filter material is arranged to retain in the first chamber (3), when the platform is rotated, cell-membrane particles which are produced when a biological cell is lysed.

3. Device according to either of claims 1 and 2, **characterised in that** associated with the first chamber (3) and/or the first channel (5) there is a retaining means which prevents or impedes the transport of a liquid sample from the first chamber (3) to the second chamber (4) when the platform is not rotating or is rotating at a speed which is below a predetermined threshold level and/or
a switching means is provided to initiate rotation of the platform at a speed which is above a predetermined threshold level as soon as a liquid sample has remained in the first chamber for a predetermined length of time.

4. Method for the lysis of biological cells, having the following steps:
- provision of a liquid containing biological cells of a single type or different types,
- provision of a device according to one of claims 1 to 3,
- introduction of a volume of the liquid into the first chamber (3) of the device,
- bringing of the liquid into contact with the reagent for lysis, in the first chamber (3), until a predetermined proportion of the cells of a selected type are lysed,
- rotation of the platform about the centre (2) of the device so that the liquid is transported from the first chamber (3) through the first channel (5) into the second chamber (4) and at least some of the membrane particles from lysed cells, and/or other solids, are retained in the first chamber (3) by the filter material.

5. Method according to claim 4, **characterised in that**, at least at times while the liquid (3) is being brought into contact with the reagent for lysis in the first chamber, the platform does not rotate or only rotates at a speed which does not allow any transport of liquid from the first chamber (3) through the first channel (5) into the second chamber (4).

6. Method according to claim 4 or 5, **characterised in that**, in the first chamber (3), the liquid can be brought into contact with the reagent for lysis for a predetermined length of time.

7. Method according to one of claims 4 to 6, **characterised in that** the platform is rotated about the centre (2) for sufficiently long, and at a speed such, that a maximum of 20% by weight of the sample liquid remains in the first chamber (3) after the rotation.

8. Assay arrangement comprising a device according to one of claims 1 to 3 and
- means for examining a sample liquid during or after withdrawal from the second chamber (4).

9. Method of carrying out an assay, comprising
- a method for the lysis of biological cells according to one of claims 4 to 7, and
- examination of the lysed sample liquid during or after withdrawal from the second chamber (4).

10. Use of a device according to one of claims 1 to 3 for the lysis of biological cells.

## Revendications

1. Dispositif de lyse de cellules biologiques comprenant :
- une plate-forme rotative, comprenant un disque plat (1) qui présente un centre (2) autour duquel la plate-forme peut tourner,
- une première chambre (3), comprenant un puits dans le disque plat (1) avec un volume compris entre 0,1 µl et 100 µl,
- une seconde chambre (4), comprenant un puits dans le disque plat (1) avec un volume compris entre 0,1 µl et 100 µl, dans laquelle la seconde chambre (4) est positionnée en direction radiale plus écartée du centre (2) que la première chambre (3),
- un premier canal (5) dans le disque plat (1) qui relie la première et la seconde chambre (3, 4) et est disposé et arrangé de telle sorte qu'un transport soutenu par la force centripète d'un fluide échantillon soit possible de la première vers la seconde chambre (3, 4) par rotation de la plate-forme autour du centre (2) ,
dans lequel la première chambre (3) comprend :
- un matériau filtrant qui est disposé dans la première chambre (3) de manière à ce que le liquide échantillon versé dans la première chambre (3) subisse par rotation de la plate-forme une force en direction du matériau filtrant et/ou depuis le matériau filtrant vers l'extérieur en direction du premier canal (5), et un réactif pour la lyse de cellules biologiques, qui est fixé sur le matériau filtrant de manière réversible.

2. Dispositif selon la revendication 1, **caractérisé en ce que** le matériau filtrant est disposé de manière à retenir dans la première chambre (3), par rotation de la plate-forme, les particules de membrane cellulaire qui sont formées lors de la lyse d'une cellule biologique.

3. Dispositif selon l'une des revendications 1 ou 2, **caractérisé en ce que** la première chambre (3) et/ou le premier canal (5) est doté d'un moyen de retenue qui empêche ou gène le transport d'un échantillon de fluide depuis la première chambre (3) vers la seconde chambre (4), lorsque la plate-forme ne tourne pas ou tourne à une vitesse inférieure à une valeur seuil prédéterminée
et/ou
un dispositif de commutation est prévu afin de provoquer une rotation de la plate-forme à une vitesse supérieure à une valeur seuil prédéterminée dès que l'échantillon de fluide a séjourné pendant une durée prédéterminée dans la première chambre (3).

4. Procédé de lyse de cellules biologiques, comprenant les étapes suivantes :
- préparation d'un fluide contenant des cellules biologiques d'un type cellulaire spécifique ou de différents types cellulaires,
- préparation d'un dispositif selon l'une des revendications 1 à 3,
- apport d'un volume du fluide dans la première chambre (3) du dispositif,
- mise en contact du fluide dans la première chambre (3) avec le réactif de lyse, jusqu'à ce qu'une part prédéterminée des cellules d'un type cellulaire choisi soit lysée,
- rotation de la plate-forme autour du centre (2) du dispositif, de sorte que le fluide soit transporté hors de la première chambre (3) par le premier canal (5) dans la seconde chambre (4) et qu'au moins une partie des particules de membrane des cellules lysées et/ou des autres solides soit maintenue dans la première chambre (3) par le matériau filtrant.

5. Procédé selon la revendication 4, **caractérisé en ce que** la plate-forme, pendant la mise en contact du fluide dans la première chambre (3) avec le réactif de lyse, ne tourne pas au moins temporairement ou ne tourne qu'à une vitesse ne permettant pas le transport du fluide hors de la première chambre (3) par le premier canal (5) dans la seconde chambre (4).

6. Procédé selon la revendication 4 ou 5, **caractérisé en ce que** le fluide, pendant une période prédéterminée, est mis en contact dans la première chambre (3) avec le réactif de lyse.

7. Procédé selon l'une des revendications 4 à 6, **caractérisé en ce que** la plate-forme tourne aussi longtemps et à une vitesse telle autour du centre (2) qu'au maximum 20 % en poids du fluide échantillon restent dans la première chambre (3) après la rotation.

8. Equipement de test comprenant un dispositif selon l'une des revendications 1 à 3 et
- un moyen pour l'étude d'un fluide échantillon dans ou après prélèvement dans la seconde chambre (4).

9. Procédé pour la réalisation d'un test comprenant
- un procédé pour la lyse de cellules biologiques selon l'une des revendications 4 à 7 et
- l'étude du fluide échantillon lysé dans ou après prélèvement dans la seconde chambre (4).

10. Utilisation d'un dispositif selon l'une des revendications 1 à 3 pour la lyse de cellules biologiques.
